# EUROPÄISCHE PATENTANMELDUNG

(11) **EP 4 450 014 A1**
(43) Veröffentlichungstag der Anmeldung: **23.10.2024**
(21) Anmeldenummer: 24170148.1
(22) Anmeldetag: 15.04.2024
(51) Int. Cl.: A61B 34/20, A61B 90/30, A61B 34/00, G06T 7/00, G06T 7/73, H04N 17/00

(54) **MEDIZINISCHE VORRICHTUNG, MEDIZINISCHES SYSTEM UND VERFAHREN ZUR ÜBERWACHUNG EINES QUELL-BILDSTROMS**

(30) Priorität: 17.04.2023 DE 102023109620; 17.04.2023 DE 102023109621
(71) Anmelder: Karl Storz SE & Co. KG, 78532 Tuttlingen (DE)
(72) Erfinder: Brüderle, Klaus, 78532 Tuttlingen (DE)

(57) **Zusammenfassung**

Medizinische Vorrichtung (10) zur Überwachung eines Quell-Bildstroms (14) mit mehreren aufeinanderfolgenden Quell-Bildern, wobei die Vorrichtung (10) aufweist: eine Verarbeitungseinrichtung (18), die dafür ausgebildet ist, den Quell-Bildstroms (14) zu empfangen und die Quell-Bilder (16) des Quell-Bildstroms (14) mit einer Linie (70) eines definierten Testmusters zu überlagern, wobei sich das Testmuster im zeitlichen Verlauf der Quell-Bilder (16) gemäß einer definierten Vorgabe fortlaufend dreht, und die derart überlagerten Quell-Bilder (16) als Sende-Bilder (22) eines Sende-Bildstroms (20) zu senden; und eine Verifikationseinrichtung (24), die dafür ausgebildet ist, den Sende-Bildstrom (20) zu empfangen, die Sende-Bilder (22) des Sende-Bildstroms (20) hinsichtlich mehrerer Bedingungen (28, 30) zu überprüfen und ein Warnsignal (26) auszugeben, falls mindestens eine Bedingung (28; 30) nicht erfüllt ist. Es werden ferner ein entsprechendes medizinisches System (1) und ein Verfahren (50) aufgezeigt.

## Beschreibung

Die Erfindung betrifft eine medizinische Vorrichtung, ein medizinisches System und ein Verfahren zur Überwachung eines Quell-Bildstroms mit mehreren aufeinanderfolgenden Quell-Bildern.

Die wichtigste Anforderung im Bereich der medizinischen Videoendoskopie ist, Benutzern einen Bildstrom der Endoskop-Kamera zur Betrachtung zur Verfügung zu stellen. Dies geschieht z.B. im Rahmen einer Untersuchung oder einer Operation, indem die Motivszenen live präsentiert oder aufgezeichnet werden. Zusätzlich werden öfter auch begleitende Bild-, Medien- oder Datenströme behandelt.

Bei der heutigen Technik betrifft die Prüfung eines Bildstroms hauptsächlich den Entwicklungsvorgang. Hierbei wird die Plausibilität einer Bildvisualisierung oder Aufzeichnung meisten humanphysiologisch vorgenommen. Konkret prüft ein Tester eine Bildstrom visuell die Aktualität, die Vollständigkeit und die Präsenz von Artefakten über einen entsprechenden Zeitraum.

Manchmal wird im Rahmen eines automatisierten Tests auch ein Testbild eingespeist und am Ziel ausgewertet. Zum Beispiel werden abwechselnd grüne und rote Bilder geschickt und deren Empfang wird geprüft. Dies geschieht in einer dedizierten Testumgebung. Für Worstcase- oder Laufzeit-Aussagen wird dieses dedizierte Testnetz hinsichtlich seiner Leistungsfähigkeit bzw. Performance modifiziert. Eine Zuverlässigkeitsvorhersage über alle unsere Systemkombinationen wird dann auf Basis dieser Ergebnisse aus der Testumgebung gemacht. Eine Überprüfung zur Laufzeit findet derzeit nicht statt.

Es ist daher eine Aufgabe eine verbesserte medizinische Vorrichtung, ein verbessertes medizinisches System und ein verbessertes Verfahren zur Überwachung eines Quell-Bildstroms mit mehreren aufeinanderfolgenden Quell-Bildern aufzuzeigen, die auch eine kontinuierliche Überwachung zur Laufzeit ermöglichen.

Die Aufgabe wird nach einem ersten Aspekt gelöst durch eine medizinische Vorrichtung zur Überwachung eines Quell-Bildstroms mit mehreren aufeinanderfolgenden Quell-Bildern, wobei die Vorrichtung aufweist:
eine Verarbeitungseinrichtung, die dafür ausgebildet ist, den Quell-Bildstrom zu empfangen und die Quell-Bilder des Quell-Bildstroms mit einer Linie eines definierten Testmusters zu überlagern, wobei sich das Testmuster im zeitlichen Verlauf der Quell-Bilder gemäß einer definierten Vorgabe fortlaufend dreht, und die derart überlagerten Quell-Bilder als Sende-Bilder eines Sende-Bildstroms zu senden;
eine Verifikationseinrichtung, die dafür ausgebildet ist, den Sende-Bildstrom zu empfangen, die Sende-Bilder des Sende-Bildstroms hinsichtlich mehrerer Bedingungen zu überprüfen und ein Warnsignal auszugeben, falls mindestens eine Bedingung nicht erfüllt ist,
wobei eine erste Bedingung ist, dass mindestens in einem definierten Prozentsatz der Sende-Bilder das definierte Testmuster identifiziert werden konnte, und eine zweite Bedingung ist, dass in diesen Sende-Bildern eine Drehung des Testmusters gemäß der definierten Vorgabe identifiziert werden konnte.

Diese Vorrichtung ermöglicht eine kontinuierliche Überprüfung, dass die Sende-Bilder fortlaufend in der richtigen Reihenfolge empfangen werden und auch eine fortlaufende Bildinformation enthalten, also nicht trotz fortlaufendem Empfang von Bildern in der richtigen Reihenfolge, diese Bilder aufgrund eines Fehlers statisch sind oder statische Bildbereiche enthalten, die eigentlich Veränderungen zeigen müssten.

Das Testmuster kann beispielsweise als Polygon, insbesondere als Dreieck, Viereck oder Stern, gewählt werden. Auch eine gerundete Form, insbesondere ein Oval, ist als Testmuster ist möglich. Selbst ein Kreis kann verwendet werden, wobei sich dieser dann nicht um seinen Mittelpunkt, sondern exzentrisch dreht. Die Linie des Testmusters kann zudem an mindestens einer Stelle unterbrochen sein, um die Orientierung des Testmusters einfacher oder zuverlässiger zu identifizieren. Das Testmuster lässt sich dynamisch in die Quell-Bilder, also das Originalmotiv, integrieren. Zudem lässt sich das Testmuster auch auf einfache Weise erzeugen.

Darüber hinaus ist das Testmuster aber auch verhältnismäßig einfach mathematisch erfassbar und auffindbar. Dies gilt auch dann, wenn die Quell-Bilder nachfolgend Veränderungen unterworfen sind, wie zum Beispiel einer Größenänderung, eine Veränderung des Seitenverhältnisses, wenn Bildteile ausgeschnitten, separiert oder zusammengeführt werden oder mit weiteren Informationen überlagert werden.

Das Testmuster wird so gewählt, dass es im Laufe der Zeit mit jedem Teil des Bildes, oder zumindest mit jedem Bildbereich von Interesse (ROI, region of interest) interagiert, konkret, überstreicht. Schließlich ist das Testmuster auch nicht nur per Bildbearbeitungsmathematik gut erfassbar, sondern auch humanvisuell einfach behandelbar oder erkennbar. Es kann auch vorteilhaft sein, dass das Testmuster genau einen Anfangspunkt und genau einen Endpunkt hat.

Die vorliegende technische Lösung bietet auch Vorteile bei mehreren Medienstrominhalten, wie Bilddatenströmen, die einem Benutzer zur Live-Ansicht oder zur komponierten Aufzeichnung gebracht werden. Hier ist es gewünscht, dass die Darstellung der Bildströme nicht nur zeitaktuell und in der richtigen Reihenfolge, sondern auch synchron zueinander erfolgt.

Zudem beginnt die Überwachung des Datenstroms, konkret, des Quell-Bilddatenstroms nicht erst wenn die Kamera, respektive der Bildgeber, das Quell-Bild geliefert hat, sondern schon bei der Motiverfassung - also vor oder wenigstens im Bildgeber selbst. Der Sende-Bildstrom wird insbesondere über ein Gerätekabel und/oder ein Netzwerk, drahtlos oder drahtgebunden, übertragen.

Werden Bilder oder Bildsegmente separiert, segmentiert und/oder wieder kombiniert, können über die definierte Form des Testmusters verschiedene Überprüfungen vorgenommen werden. An den Grenzen von Bildsegmenten können die Segmentgrenzverläufe geprüft werden. Wenn die Linie des Testmusters kontinuierlich verläuft, also ohne Absätze oder Stufen, sind die Bildsegmente korrekt positioniert.

Dabei können gleichzeitig auch Größenverhältnisse überprüft oder angepasst werden. Wenn die Linie des Testmusters kontinuierlich verläuft, besteht eine hohe Sicherheit, dass auch die Größenverhältnisse mehrerer Segmente zueinander passen.

Es wird hier also die Interaktion von Originalmotiv und die Einprägung eines Testmusters auf das Quell-Bild vorgeschlagen. Die Einprägung sollte möglichst am Ursprung des Bildstroms erfolgen. Es kann aber auch sinnvoll sein, dies erst an einem anderen Punkt des Bildstrompfades vorzunehmen, wobei die Überprüfung dann aber auch nur ab diesem Punkt ermöglicht wird. Die Auswertung oder Verifikation sollte möglichst am Endpunkt stattfinden. Sie kann grundsätzlich auch vorher stattfinden, doch ist der nachfolgende Teil des Pfads dann nicht überprüfbar.

Solange das Testmuster sichtbar eingeprägt wird, wird für eine Evaluierung zur Laufzeit vorgeschlagen, dass diese Einprägung des Testmusters nur zum Setup- oder Einschaltzeitpunkt aktiv ist. So kann eine Überprüfung vor dem eigentlichen Einsatz durchgeführt werden, sozusagen ein Referenzvorgang. Alternativ oder zusätzlich ist es möglich, eine solche Überprüfung nach Abschluss des eigentlichen Einsatzes durchzuführen, insbesondere zu Nachweis- oder Dokumentationszwecken.

Die hier aufgezeigte Vorgehensweise bietet eine Vorrichtung für ein allgemeines Prüfverfahren und ein Prüfverfahren als solches für alle Arten eines Bildstromtransports. Es kann dabei bevorzugt als Prüfverfahren für Entwicklungs- und Laufzeitumgebungen eingesetzt werden. Das Prüfverfahren kann dabei völlig automatisiert durchgeführt werden, einschließlich der Verifikation und einer Erkennung von Fehlern. Eine Dokumentation kann dabei einmalig, insbesondere als Einzelnachweis, oder wiederholt, insbesondere als Qualitätsnachweis, durchgeführt werden.

Damit ist die Aufgabe vollständig gelöst.

Bei einer bevorzugten Ausgestaltung weist das Testmuster eine Spiralform auf.

Aufgrund der mathematisch eindeutigen Beschreibung der Spiralform ist eine weitere vorteilhafte Auswertung möglich. Dabei sein angenommen, dass zu einem späteren Zeitpunkt nur ein Bildausschnitt eines Sende-Bilds zur Verfügung steht, in dem einer oder mehrere Abschnitte der Spiralform enthalten sind. Diese Abschnitte können als Spiralliniensegmente bezeichnet werden. Die mathematische Beschreibung der Spiralform erlaubt es in der Umkehr, aus einem dieser Spiralliniensegmente den Mittelpunkt des Sende-Bilds zu ermitteln, zu dem der Bildausschnitt gehört.

Für die Überprüfung könnte man im Sende-Bild eine Gerade durch den Ursprungspunkt der Spiralform ziehen, die Schnittpunkte mit der Spirale erkennen und mathematisch prüfen, ob diese plausibel sind, sprich, ob diese Schnittpunkte der definierten Spiralform entsprechen.

Die Spiralform unterstützt zudem eine Zwei-Faktoren-Berechnung und bleibt hinsichtlich der üblichen Bildtransport- oder Bildkomprimierungsverfahren im Wesentlichen unbeeinflusst, so dass die Linie in Spiralform auch über längere Verarbeitungs- und Übergabeprozesse identifizierbar bleibt, wie zum Beispiel auch eine Komprimierung (zum Beispiel als h264 oder MJPEG).

Bei einer bevorzugten Ausgestaltung ist die Spiralform eine archimedische Spirale.

Die archimedische Spirale lässt sich im kartesischen Koordinatensystem mit den Formeln x = k * phi * cos(phi) und y = k * phi * sin(phi) beschreiben, wobei phi als Winkel verstanden werden kann und k ein Streckungs- bzw. Stauchungsfaktor für die Spirale ist. Umgekehrt gilt dann auch phi = atan(y / x). Die archimedische Spirale hat die Eigenschaft, dass jede Gerade, die durch ihren Ursprungspunkt verläuft, die Spirale mehrfach schneidet, wobei dieser Schnittpunkt entlang die Gerade gleichweit voneinander entfernt sind. Da der Streckungs- bzw. Stauchungsfaktor k hier für die x- und die y-Koordinate gleich ist, kann auch die Bedingung überprüft werden, dass die Spiralform tatsächlich kreisrund und nicht elliptisch geformt ist. Wird statt einer kreisrunden Spiralform eine elliptische Spiralform entdeckt, spricht dies für eine Stauchung oder Streckung des Sende-Bilds.

Bei einer bevorzugten Ausgestaltung ist die Linie in einer Kontrastfarbe ausgeführt, die in den Quell-Bildern nicht oder nur statistisch vernachlässigbar aufzufinden ist.

Diese Ausgestaltung macht es besonders einfach, die Linie der Spiralform für die Überprüfung aufzufinden. Zu den Kontrastfarben zählen insbesondere weiß (RGB 255,255,255) und schwarz (RGB 0,0,0).

Bei einer bevorzugten Ausgestaltung liegt ein Ursprungspunkt der Spiralform in der Mitte der Quell-Bilder.

Diese Ausgestaltung ermöglicht die Überprüfung einer weiteren Bedingung, dass die Spirale in der Bildmitte enden muss.

Bei einer bevorzugten Ausgestaltung wird zu jedem Sende-Bild des Sende-Bildstroms eine Sicherungs-Bildinformation gespeichert, die die Bildinformation aus dem jeweiligen Quell-Bild enthält, die von der Linie des Testmusters überlagert wird.

Diese Ausgestaltung stellt sicher, dass die von dem Testmuster überlagerte Bildinformation nicht verloren geht. Die Sicherungs-Bildinformation wird dabei bevorzugt in einem Randbereich des Sende-Bilds positioniert oder als Meta-Information zusammen mit dem jeweiligen Sende-Bild gesendet. Die Sicherungs-Bildinformation kann dabei konkret die Pixel-Information entlang der Spiralform enthalten oder als Maske ausgestaltet sein.

Bei einer bevorzugten Ausgestaltung weist die Vorrichtung ferner eine Wiederherstellungsvorrichtung auf, die dafür ausgebildet ist, die Linie des Testmusters mit der zugehörigen Sicherungs-Bildinformation im Sende-Bild wiederherzustellen und die derart wiederhergestellten Sende-Bilder als Präsentations-Bilder eines Präsentations-Bildstroms auszugeben.

Diese Ausgestaltung verringert oder beseitigt die Wahrnehmbarkeit des überlagerten Testmusters bei einem Betrachter. Wurde die Sicherungs-Bildinformation als Maske hinterlegt, so kann das jeweilige Sende-Bild mit seiner jeweiligen Sicherungs-Bildinformation auf einfache Weise zu einem Präsentations-Bild kombiniert werden. Dies kann insbesondere nach der Überprüfung eines Sende-Bilds erfolgen, aber auch parallel zu dieser Überprüfung.

Bei einer bevorzugten Ausgestaltung weist das Testmuster zusätzlich eine gerade Strecke auf, die von einem Ursprungspunkt oder Mittepunkt des Testmusters radial nach außen führt.

Bei dieser Ausgestaltung visualisiert die gerade Strecke in der Art eines Zeigers den Fortschritt im Bildstrom besonders deutlich. Eine gleichmäßig fortschreitende Rotation des Zeigers deutet eine korrekte Abfolge und Geschwindigkeit der Sende-Bilder an. Sollen mehrere Bildströme im Hinblick auf eine gegenseitige Synchronität überprüft werden, so können einfach die Zeiger in den Bildströmen miteinander hinsichtlich ihres Winkels vergleichen werden. Wird eine Abweichung festgestellt, so kann durch ein Verlangsamen, Beschleunigen oder ein Springen in einem der Bilderströme die Synchronität wieder hergestellt werden,

Bei einer bevorzugten Ausgestaltung enthält die definierte Vorgabe, dass sich die Spirale nach innen dreht.

Wie bereits erläutert, rotiert die Spirale während eines Verifikationsintervalls. Über diese Rotation ist eine gewisse Bilddynamik gegeben. Es wird als geeignet angesehen, dass sich die Spirale nach innen dreht, also im Uhrzeigersinn.

Bei einer bevorzugten Ausgestaltung weisen die mehreren Bedingungen mindestens eine weitere Bedingung auf: die Linie des Testmusters ist in einem definierten weiteren Prozentsatz vollständig im Sende-Bild identifizierbar.

Bei dieser Ausgestaltung ist es möglich zu überprüfen, ob möglicherweise Kompressionsverluste die Bildqualität verschlechtern.

Bei einer bevorzugten Ausgestaltung weist die Vorrichtung ferner eine Ausgabevorrichtung auf, die dafür ausgebildet ist, das Warnsignal visuell, akustisch und/oder haptisch erfassbar auszugeben.

Bei dieser Ausgestaltung erhält der Benutzer einen Hinweis, wenn der Ist-Sende-Bildstrom von dem erwarteten Soll-Sende-Bildstrom abweicht. Der Benutzer ist dann dafür sensibilisiert, dass Ist-Sende-Bildstrom evtl. nicht das zeigt, was der Benutzer bei einer idealen, fehlerfreien Übertragung erwarten würde. Das Warnsignal kann auch Informationen über die Art der Warnung, sprich, über die Art und Schwere, der nicht erfüllten Bedingung oder Bedingungen enthalten. Es ist dann möglich, dass der Benutzer auswählen kann, ob und wie er in den jeweiligen Abweichungen vom gewünschten Soll-Sende-Bildstrom benachrichtigt werden will.

Bei einer bevorzugten Ausgestaltung ist die Verarbeitungseinrichtung ferner dazu ausgebildet, während definierter Intervalle die Sende-Bilder zu invertieren.

Diese Ausgestaltung enthält eine weitere Bilddynamik, die in den Sende-Bildern überprüft werden kann, um den Sende-Bildstrom zu überwachen. Kann die Verifikationseinrichtung solche Wechsel zwischen aktivierter Invertierung und deaktivierter Invertierung nicht oder nicht in einem Abstand der definierten Intervalle erkennen, kann dies als mögliche Fehlersituation signalisiert werden.

Gemäß einem zweiten Aspekt wird die Aufgabe gelöst durch ein medizinisches System zur Überwachung eines Bildstroms mit mehreren aufeinanderfolgenden Bildern aufweisend einen Bildgeber zur Erzeugung des Quell-Bildstroms, einer Vorrichtung nach einem der vorhergehenden Ansprüche, und einer Anzeigevorrichtung, die dafür ausgebildet ist, den Sende-Bildstrom auszugeben und das Warnsignal wiederzugeben, wobei die Verarbeitungseinrichtung insbesondere in den Bildgeber integriert ist.

Gemäß einem zweiten Aspekt wird die Aufgabe gelöst durch ein Verfahren zur Überwachung eines Quell-Bildstroms mit mehreren aufeinanderfolgenden Quell-Bildern, das Verfahren mit den Schritten:
Empfangen des Quell-Bildstroms;
Überlagern der Quell-Bilder des Quell-Bildstroms mit einer Linie eines definierten Testmusters, wobei sich das Testmuster im zeitlichen Verlauf der Quell-Bilder gemäß einer definierten Vorgabe fortlaufend dreht;
Senden der derart überlagerten Quell-Bilder als Sende-Bilder eines Sende-Bildstroms;
Empfangen des Sende-Bildstroms;
Überprüfen der Sende-Bilder des Sende-Bildstroms hinsichtlich mehrerer Bedingungen; und
Ausgeben eines Warnsignals, falls mindestens eine Bedingung nicht erfüllt ist,
wobei eine erste Bedingung ist, dass mindestens in einem definierten Prozentsatz der Sende-Bilder das definierte Testmuster identifiziert werden konnte, und eine zweite Bedingung ist, dass in diesen Sende-Bildern eine Drehung des Testmusters gemäß der definierten Vorgabe identifiziert werden konnte.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Ausführungsform eines medizinischen Systems zur Überwachung eines Quell-Bildstroms mit mehreren aufeinanderfolgenden Quell-Bildern mit einer Ausführungsform einer medizinischen Vorrichtung zur Überwachung eines Quell-Bildstroms mit mehreren aufeinanderfolgenden Quell-Bildern;
- Fig. 2: eine Ausführungsform eines Verfahrens zur Überwachung eines Quell-Bildstroms mit mehreren aufeinanderfolgenden Quell-Bildern;
- Fig. 3: eine Ausführungsform eines Quell-Bilds;
- Fig. 4: eine Ausführungsform eines Testmusters als Spiralform;
- Fig. 5: eine Ausführungsform eines Sende-Bilds, das sich aus der Überlagerung der Quell-Bilds aus Fig. 3 und der Spiralform aus Fig. 4 ergibt.
- Fig. 6: eine Ausführungsform eines Sende-Bilds, bei der sich ausgehend von Fig. 5 die Spiralform gedreht hat;
- Fig. 7: eine Ausführungsform eines Sende-Bilds, bei der sich ausgehend von Fig. 5 die Spiralform weitergedreht hat; und
- Fig. 8: eine Ausführungsform eines Sende-Bilds, bei der sich ausgehend von Fig. 5 die Spiralform nochmals weitergedreht hat.

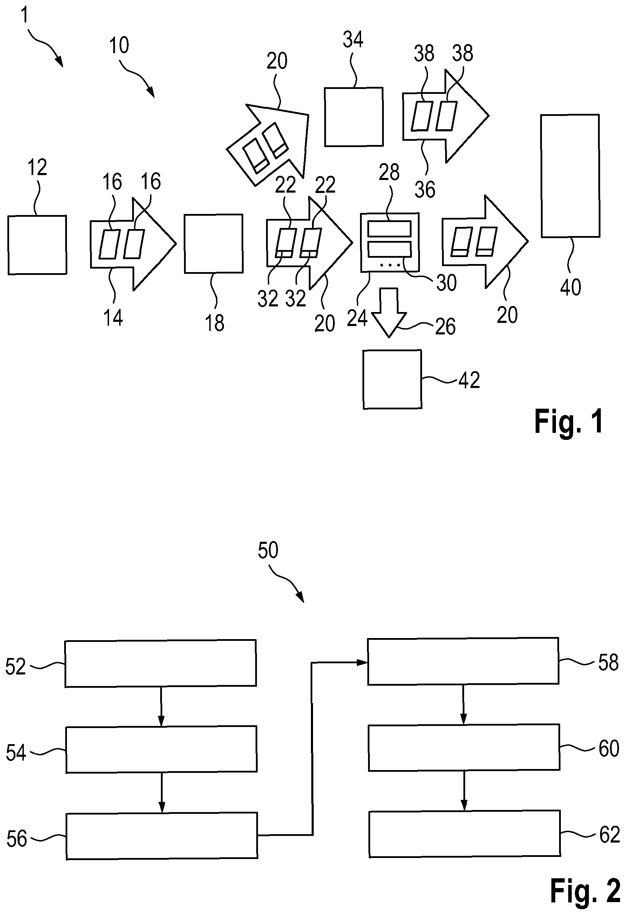

Fig. 1 zeigt ein medizinisches System 1 zur Überwachung eines Quell-Bildstroms 14 mit mehreren aufeinanderfolgenden Quell-Bildern 16 aufweisend einen Bildgeber 12 zur Erzeugung des Quell-Bildstroms 14, einer medizinischen Vorrichtung 10 zur Überwachung eines Quell-Bildstroms 14 mit mehreren aufeinanderfolgenden Quell-Bildern 16, und einer Anzeigevorrichtung 40, die dafür ausgebildet ist, den Sende-Bildstrom auszugeben und das Warnsignal wiederzugeben.

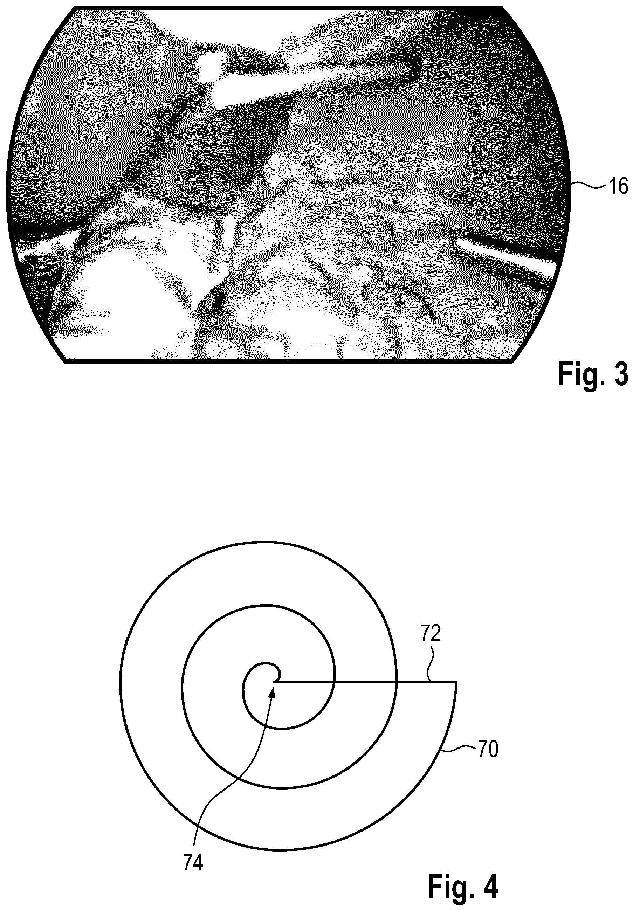

Die Vorrichtung 10 weist verschiedene Elemente auf, darunter eine Verarbeitungseinrichtung 18, die dafür ausgebildet ist, den Quell-Bildstrom 14 zu empfangen und die Quell-Bilder 16 des Quell-Bildstroms 14 mit einer Linie 70 (siehe Fig. 4) eines definierten Testmusters zu überlagern, wobei sich das Testmuster im zeitlichen Verlauf der Quell-Bilder 16 gemäß einer definierten Vorgabe fortlaufend dreht, und die derart überlagerten Quell-Bilder 16 als Sende-Bilder 22 eines Sende-Bildstroms 20 zu senden. Die Verarbeitungseinrichtung 18 ist insbesondere in den Bildgeber 12 integriert. Das Testmuster ist hier eine definierte Spiralform.

Die Vorrichtung 10 weist ferner eine Verifikationseinrichtung 24, die dafür ausgebildet ist, den Sende-Bildstrom 22 zu empfangen, die Sende-Bilder 22 des Sende-Bildstroms 20 hinsichtlich mehrerer Bedingungen 28, 30 zu überprüfen und ein Warnsignal 26 auszugeben, falls mindestens eine Bedingung 28, 30 nicht erfüllt ist.

Zu den mehreren Bedingungen 28, 30 gehört eine erste Bedingung 28, dass mindestens in einem definierten Prozentsatz der Sende-Bilder 22 die definierte Spiralform identifiziert werden konnte, und eine zweite Bedingung 30 ist, dass in diesen Sende-Bildern 22 eine Drehung der Spiralform gemäß der definierten Vorgabe identifiziert werden konnte.

Bei dieser Ausführungsform wird zu jedem Sende-Bild 22 des Sende-Bildstroms 20 eine Sicherungs-Bildinformation 32 gespeichert wird, die die Bildinformation aus dem jeweiligen Quell-Bild 16 enthält, die von der Linie 70 der Spiralform überlagert wird.

Die Sicherungs-Bildinformation 32 wird von einer Wiederherstellungsvorrichtung 34 genutzt, die dafür ausgebildet ist, die Linie 70 der Spiralform mit der zugehörigen Sicherungs-Bildinformation 32 im Sende-Bild 22 wiederherzustellen und die derart wiederhergestellten Sende-Bilder 22 als Präsentations-Bilder 38 eines Präsentations-Bildstroms 36 auszugeben.

Die Vorrichtung 10 weist ferner eine Ausgabevorrichtung 42 auf, die dafür ausgebildet ist, das Warnsignal 26 visuell, akustisch und/oder haptisch erfassbar auszugeben. Zudem ist die Verarbeitungseinrichtung 18 bei dieser Ausführungsform ferner dazu ausgebildet, während definierter Intervalle die Sende-Bilder 22 zu invertieren.

Fig. 2 zeigt ein Verfahren 50 zur Überwachung eines Quell-Bildstroms 14 mit mehreren aufeinanderfolgenden Quell-Bildern 16. Die Ausführungsform des gezeigten Verfahrens 50 beginnt mit dem Empfangen 52 des Quell-Bildstroms 14, gefolgt von einem Überlagern 54 der Quell-Bilder 14 des Quell-Bildstroms 16 mit einer Linie 70 in einer definierten Spiralform, wobei sich die Spiralform im zeitlichen Verlauf der Quell-Bilder 16 gemäß einer definierten Vorgabe fortlaufend dreht.

Das Verfahren 50 fährt mit dem Senden 56 der derart überlagerten Quell-Bilder 16 als Sende-Bilder 22 des Sende-Bildstroms 20, dem Empfangen 58 des Sende-Bildstroms 20 und dem Überprüfen 60 der Sende-Bilder 22 des Sende-Bildstroms 20 hinsichtlich mehrerer Bedingungen 28, 30. Falls mindestens eine Bedingung 28, 30 nicht erfüllt ist, erfolgt das Ausgeben 62 eines Warnsignals 26.

Auch hier gilt wieder: Eine erste Bedingung 28 ist, dass mindestens in einem definierten Prozentsatz der Sende-Bilder die definierte Spiralform identifiziert werden konnte, und eine zweite Bedingung 30 ist, dass in diesen Sende-Bildern 22 eine Drehung der Spiralform gemäß der definierten Vorgabe identifiziert werden konnte.

Fig. 3 zeigt eine Ausführungsform eines Quell-Bilds 16.

Fig. 4 zeigt eine Ausführungsform eines Testmusters, hier eine Spiralform, als Linie 70. Die Spiralform ist hier eine archimedische Spirale. Die Linie 70 ist in einer Kontrastfarbe ausgeführt, hier Schwarz, die in den Quell-Bildern 16 nicht oder nur statistisch vernachlässigbar aufzufinden ist. Die Spiralform weist zusätzlich eine gerade Strecke 72 auf, die von einem Ursprungspunkt 74 der Spiralform radial nach außen führt.

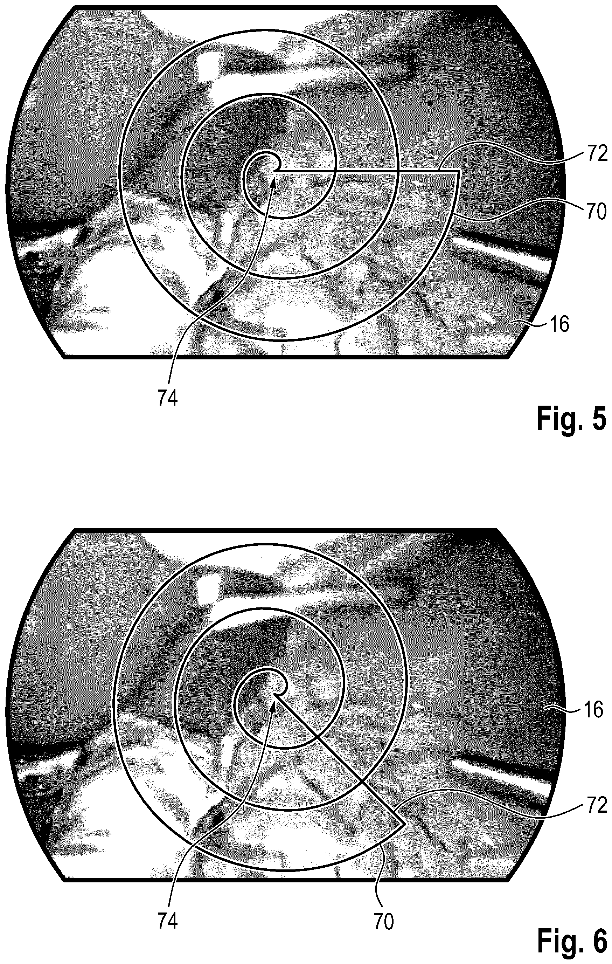

Fig. 5 zeigt eine Ausführungsform eines Sende-Bilds 22, das sich aus der Überlagerung der Quell-Bilds 16 aus Fig. 3 und der Linie 70 in Spiralform aus Fig. 4 ergibt.

Fig. 6 zeigt eine Ausführungsform eines Sende-Bilds 22, bei der sich ausgehend von Fig. 5 die Spiralform gedreht hat.

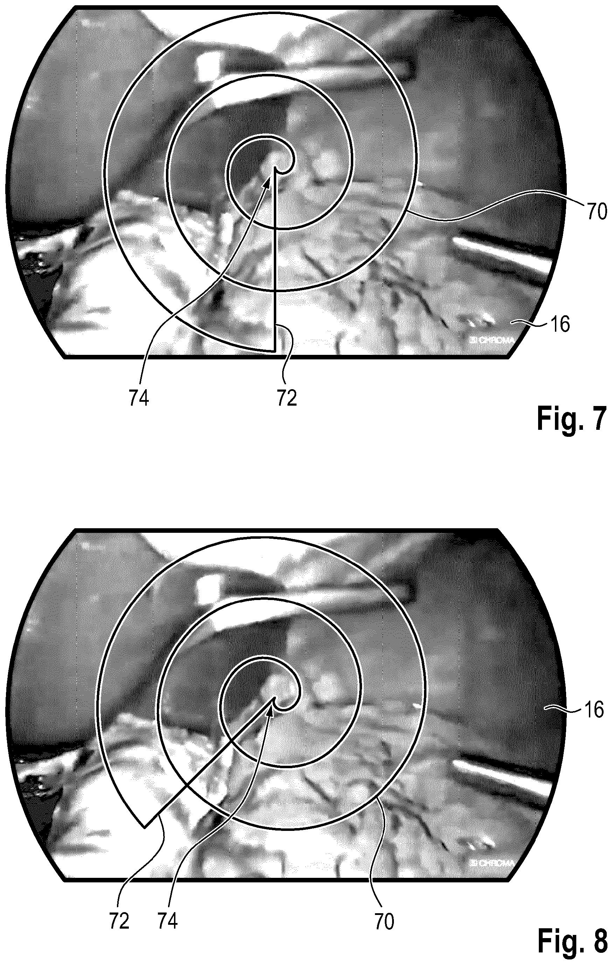

Fig. 7 zeigt eine Ausführungsform eines Sende-Bilds 22, bei der sich ausgehend von Fig. 5 die Spiralform weitergedreht hat; und

Fig. 8 zeigt eine Ausführungsform eines Sende-Bilds 22, bei der sich ausgehend von Fig. 5 die Spiralform nochmals weitergedreht hat.

## Patentansprüche

1. Medizinische Vorrichtung (10) zur Überwachung eines Quell-Bildstroms (14) mit mehreren aufeinanderfolgenden Quell-Bildern (16), wobei die Vorrichtung (10) aufweist:
eine Verarbeitungseinrichtung (18), die dafür ausgebildet ist, den Quell-Bildstrom (14) zu empfangen und die Quell-Bilder (16) des Quell-Bildstroms (14) mit einer Linie (70) eines definierten Testmusters zu überlagern, wobei sich das Testmuster im zeitlichen Verlauf der Quell-Bilder (16) gemäß einer definierten Vorgabe fortlaufend dreht, und die derart überlagerten Quell-Bilder (16) als Sende-Bilder (22) eines Sende-Bildstroms (20) zu senden;
eine Verifikationseinrichtung (24), die dafür ausgebildet ist, den Sende-Bildstrom (20) zu empfangen, die Sende-Bilder (22) des Sende-Bildstroms (20) hinsichtlich mehrerer Bedingungen (28, 30) zu überprüfen und ein Warnsignal (26) auszugeben, falls mindestens eine Bedingung (28; 30) nicht erfüllt ist,
wobei eine erste Bedingung (28) ist, dass mindestens in einem definierten Prozentsatz der Sende-Bilder (22) das definierte Testmuster identifiziert werden konnte, und eine zweite Bedingung (30) ist, dass in diesen Sende-Bildern (22) eine Drehung des Testmusters gemäß der definierten Vorgabe identifiziert werden konnte.

2. Vorrichtung (10) nach Anspruch 1, wobei das Testmuster eine Spiralform aufweist.

3. Vorrichtung (10) nach Anspruch 2, wobei die Spiralform eine archimedische Spirale ist.

4. Vorrichtung (10) nach einem der vorhergehenden Ansprüche, wobei die Linie (70) in einer Kontrastfarbe ausgeführt ist, die in den Quell-Bildern (16) nicht oder nur statistisch vernachlässigbar aufzufinden ist.

5. Vorrichtung (10) nach einem der Ansprüche 2 bis 4, wobei ein Ursprungspunkt (74) der Spiralform in der Mitte der Quell-Bilder (16) liegt.

6. Vorrichtung (10) nach einem der vorhergehenden Ansprüche, wobei zu jedem Sende-Bild (22) des Sende-Bildstroms (20) eine Sicherungs-Bildinformation (32) gespeichert wird, die die Bildinformation aus dem jeweiligen Quell-Bild (16) enthält, die von der Linie (70) des Testmusters überlagert wird.

7. Vorrichtung (10) nach Anspruch 6, ferner mit einer Wiederherstellungsvorrichtung (34), die dafür ausgebildet ist, die Linie (70) des Testmusters mit der zugehörigen Sicherungs-Bildinformation (32) im Sende-Bild (22) wiederherzustellen und die derart wiederhergestellten Sende-Bilder (22) als Präsentations-Bilder (38) eines Präsentations-Bildstroms (36) auszugeben.

8. Vorrichtung (10) nach einem der vorhergehenden Ansprüche, wobei das Testmuster zusätzlich eine gerade Strecke (72) aufweist, die von einem Ursprungspunkt (74) oder Mittelpunkt des Testmusters radial nach außen führt.

9. Vorrichtung (10) nach einem der Ansprüche 2 bis 8, wobei die definierte Vorgabe enthält, dass sich die Spirale nach innen dreht.

10. Vorrichtung (10) nach einem der vorhergehenden Ansprüche, wobei die mehreren Bedingungen (28, 30) mindestens eine weitere Bedingung aufweisen: die Linie des Testmusters ist in einem definierten weiteren Prozentsatz vollständig im Sende-Bild identifizierbar.

11. Vorrichtung (10) nach einem der vorhergehenden Ansprüche, ferner mit einer Ausgabevorrichtung (42), die dafür ausgebildet ist, das Warnsignal (26) visuell, akustisch und/oder haptisch erfassbar auszugeben.

12. Vorrichtung (10) nach einem der vorhergehenden Ansprüche, wobei die Verarbeitungseinrichtung (18) ferner dazu ausgebildet ist, während definierter Intervalle die Sende-Bilder (22) zu invertieren.

13. Medizinisches System (1) zur Überwachung eines Quell-Bildstroms (14) mit mehreren aufeinanderfolgenden Quell-Bildern (16) aufweisend einen Bildgeber (12) zur Erzeugung des Quell-Bildstroms (14), einer Vorrichtung (10) nach einem der vorhergehenden Ansprüche, und einer Anzeigevorrichtung (40), die dafür ausgebildet ist, den Sende-Bildstrom (20) auszugeben und das Warnsignal (26) wiederzugeben, wobei die Verarbeitungseinrichtung (18) insbesondere in den Bildgeber (12) integriert ist.

14. Verfahren (50) zur Überwachung eines Quell-Bildstroms (14) mit mehreren aufeinanderfolgenden Quell-Bildern (16), das Verfahren mit den Schritten:
Empfangen (52) des Quell-Bildstroms (14);
Überlagern (54) der Quell-Bilder (16) des Quell-Bildstroms (14) mit einer Linie eines definierten Testmusters, wobei sich das Testmuster im zeitlichen Verlauf der Quell-Bilder (16) gemäß einer definierten Vorgabe fortlaufend dreht;
Senden (56) der derart überlagerten Quell-Bilder (16) als Sende-Bilder (22) eines Sende-Bildstroms;
Empfangen (58) des Sende-Bildstroms;
Überprüfen (60) der Sende-Bilder (22) des Sende-Bildstroms (20) hinsichtlich mehrerer Bedingungen (28, 30); und
Ausgeben (62) eines Warnsignals, falls mindestens eine Bedingung (28; 30) nicht erfüllt ist,
wobei eine erste Bedingung (28) ist, dass mindestens in einem definierten Prozentsatz der Sende-Bilder (22) das definierte Testmuster identifiziert werden konnte, und eine zweite Bedingung (30) ist, dass in diesen Sende-Bildern (22) eine Drehung des Testmusters gemäß der definierten Vorgabe identifiziert werden konnte.

15. Verfahren nach Anspruch 14, wobei das Testmuster eine Spiralform aufweist, die insbesondere eine archimedische Spirale ist.
